# EUROPEAN PATENT SPECIFICATION

(11) **EP 2 777 631 B1**
(45) Date of publication and mention of the grant of the patent: **29.11.2017**
(21) Application number: 14159365.7
(22) Date of filing: 13.03.2014
(51) Int. Cl.: A61F 2/44, A61F 2/46, A61F 2/30, A61F 2/28, A61F 2/48

(54) **Selectively expanding spine cage with enhanced bone graft infusion**
Selektiv dehnbarer Wirbelsäulenkäfig mit verbesserter Knochenimplantatinfusion
Cage vertébrale à expansion sélective avec une meilleure infusion de greffe osseuse

(30) Priority: 13.03.2013 US 201313799047
(43) Date of publication of application: 17.09.2014
(60) Divisional application: 17197747.3
(73) Proprietor: Howmedica Osteonics Corp., Mahwah, NJ 07430 (US)
(72) Inventor: Shulock, Damien J., Redwood City, CA California 94063 (US); Ashley, John E., Redwood City, CA California 94063 (US); Grotz, Thomas, Redwood City, CA California 94063 (US); Pretti, Rudy, Redwood City, CA California 94063 (US)
(74) Representative: Gill Jennings & Every LLP

(56) References cited:
- WO-A1-2010/074704
- WO-A2-2008/039811
- WO-A2-2008/121251

## Description

### FIELD OF THE INVENTION

The present invention generally relates to medical devices for stabilizing the vertebral motion segment. More particularly, the field of the invention relates to a remotely activated, hydraulically controllable, selectively expanding cage (SEC) and method of insertion for providing controlled spinal correction in three dimensions for improved spinal intervertebral body distraction and fusion.

### BACKGROUND

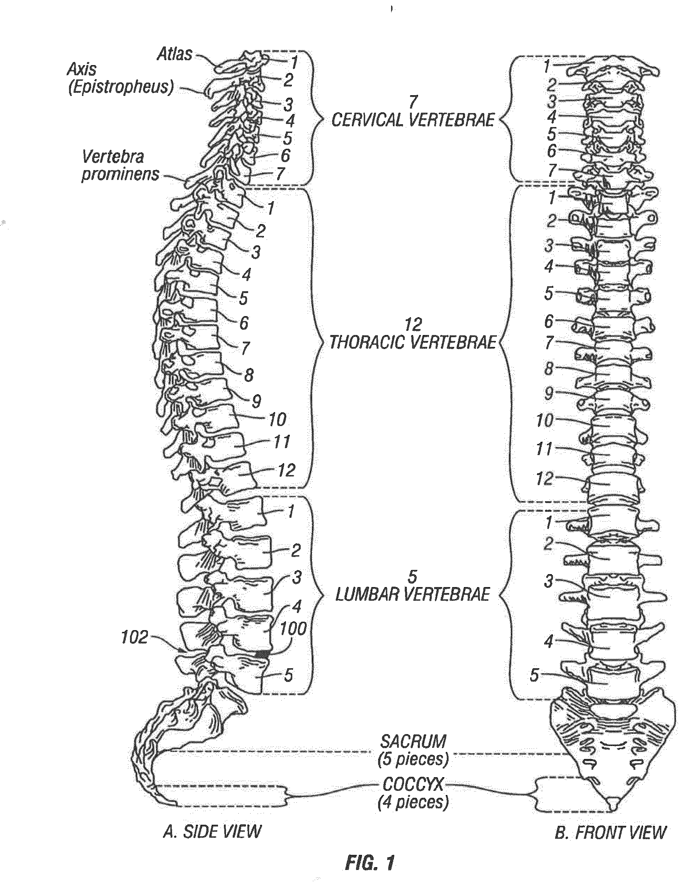

Conventional spine cages or implants are typically characterized by a kidney bean-shaped body comprising a hydroxyapatite-coated surface provided on the exterior surface for contact with adjacent vertebral segments or endplates which are shown in FIG. 1. A conventional spine cage is typically inserted in tandem posteriorly through the neuroforamen of the distracted spine after a trial implant creates a pathway.

Such existing devices for interbody stabilization have important and significant limitations. These limitations include an inability to expand and distract the endplates. Current devices for interbody stabilization include static spacers composed of titanium, PEEK, and high performance thermoplastic polymer produced by VICTREX, (Victrex USA Inc, 3A Caledon Court, Greenville, SC 29615), carbon fiber, or resorbable polymers. Current interbody spacers do not maintain interbody lordosis and can contribute to the formation of a straight or even kyphotic segment and the clinical problem of "flatback syndrome." Separation of the endplates increases space available for the neural elements, specifically the neural foramen. Existing static cages do not reliably improve space for the neural elements. Therefore, what is needed is an expanding cage that will increase space for the neural elements posteriorly between the vertebral bodies, or at least maintain the natural bone contours to avoid neuropraxia (nerve stretch) or encroachment.

Another problem with conventional devices of interbody stabilization includes poor interface between bone and biomaterial. Conventional static interbody spacers form a weak interface between bone and biomaterial. Although the surface of such implants is typically provided with a series of ridges or coated with hydroxyapetite, the ridges may be in parallel with applied horizontal vectors or side-to-side motion. That is, the ridges or coatings offer little resistance to movement applied to either side of the endplates. Thus, nonunion is common in allograft, titanium and polymer spacers, due to motion between the implant and host bone. Conventional devices typically do not expand between adjacent vertebrae.

Therefore, what is needed is a way to expand an implant to develop immediate fixation forces that can exceed the ultimate strength at healing. Such an expandable implant ideally will maximize stability of the interface and enhance stable fixation. The immediate fixation of such an expandable interbody implant advantageously will provide stability that is similar to that achieved at the time of healing. Such an implant would have valuable implications in enhancing early postoperative rehabilitation for the patient.

Another problem of conventional interbody spacers is their large diameter requiring wide exposure. Existing devices used for interbody spacers include structural allograft, threaded cages, cylindrical cages, and boomerang-shaped cages. Conventional devices have significant limitation with regard to safety and efficacy. Regarding safety of the interbody spacers, injury to neural elements may occur with placement from an anterior or posterior approach. A conventional spine cage lacks the ability to expand, diminishing its fixation capabilities.

The risks to neural elements are primarily due to the disparity between the large size of the cage required to adequately support the interbody space, and the small space available for insertion of the device, especially when placed from a posterior or transforaminal approach. Existing boomerang cages are shaped like a partially flattened kidney bean. Their implantation requires a wide exposure and potential compromise of vascular and neural structures, both because of their inability to enter small and become larger, and due to the fact that their insertion requires mechanical manipulation during insertion and expanding of the implant. Once current boomerang implants are prepared for insertion via a trial spacer to make a pathway toward the anterior spinal column, the existing static cage is shoved toward the end point with the hope that it will reach a desired anatomic destination. Given the proximity of nerve roots and vascular structures to the insertion site, and the solid, relatively large size of conventional devices, such constraints predispose a patient to foraminal (nerve passage site) encroachment, and possible neural and vascular injury.

Therefore, what is needed is a minimally invasive expanding spine cage that is capable of insertion with minimal invasion into a smaller aperture. Such a minimally invasive spine cage advantageously could be expanded with completely positional control or adjustment in three dimensions by hydraulic force application through a connected thin, pliable hydraulic line. The thin hydraulic line would take the place of rigid insertional tools, thereby completely preventing trauma to delicate nerve endings and nerve roots about the spinal column. Due to the significant mechanical leverage developed by a hydraulic control system, the same expanding cage could advantageously be inserted by a minimally-sized insertion guiding rod tool capable of directing the cage through the transforaminal approach to a predetermined destination, also with reduced risk of trauma to nerve roots. That is, the mechanical advantage is provided by a hydraulic control system controlled by the physician external to the patient.

The minimally-sized insertion tool could house multiple hydraulic lines for precise insertion and expansion of the cage, and simply detached from the expanded cage after insertion. It is noted that in such a hydraulic system, a smaller, thinner line advantageously also increases the pounds per inch of adjusting force necessary to achieve proper expansion of the implant (as opposed to a manually powered or manipulated surgical tool) that must apply force directly at the intervention site. That is, for a true minimally-invasive approach to spinal implant surgery what is needed is an apparatus and method for providing the significant amount of force necessary to properly expand and adjust the cage against the vertebral endplates, safely away from the intervention site.

What is also needed is a smaller expanding spine cage that is easier to operatively insert into a patient with minimal surgical trauma in contrast to conventional, relatively large devices that create the needless trauma to nerve roots in the confined space of the vertebral region.

Existing interbody implants have limited space available for bone graft. Adequate bone graft or bone graft substitute is critical for a solid interbody arthrodesis. It would be desirable to provide an expandable interbody cage that will permit a large volume of bone graft material to be placed within the cage and around it, to fill the intervertebral space. Additionally, conventional interbody implants lack the ability to stabilize endplates completely and prevent them from moving. Therefore, what is also needed is an expanding spine cage wherein the vertebral end plates are subject to forces that both distract them apart, and hold them from moving. Such an interbody cage would be capable of stabilization of the motion segment, thereby reducing micromotion, and discouraging the pseudoarthrosis (incomplete fusion) and pain.

Ideally, what is needed is a spine cage or implant that is capable of increasing its expansion in width anteriorly to open like a clam, spreading to a calculated degree. Furthermore, what is needed is a spine cage that can adjust the amount of not only overall anterior expansion, but also medial and lateral variable expansion so that both the normal lordotic curve is maintained, and adjustments can be made for scoliosis or bone defects. Such a spine cage or implant would permit restoration of normal spinal alignment after surgery and hold the spine segments together rigidly, mechanically, until healing occurs.

What is also needed is an expanding cage or implant that is capable of holding the vertebral or joint sections with increased pullout strength to minimize the chance of implant fixation loss during the period when the implant is becoming incorporated into the arthrodesis bone block.

It would also be desirable if such a cage could expand anteriorly away from the neural structures and along the axis of the anterior spinal column, rather than uniformly which would take up more space inside the vertebral body surfaces. WO 2008/121251 discloses an apparatus for providing spinal correction, comprising: an implant body configured and dimensioned for placement in an intervertebral space, said body defining a central cavity extending through said body configured to receive bone graft material and communicate with the intervertebral space for infusion of the graft material into the intervertebral space when placed therein, and first and second extendable members mounted on said body, one each disposed on an opposite side of said central cavity, said members extendable from a first unexpanded height and to at least one expanded height; and a bone graft material supply port disposed on the implant body, said port configured for attachment of a bone graft material supply line; and a plate with a bone engaging surface mounted on said first and second extendable members, said plate defining an opening aligned with the central cavity for passage there through of bone graft material from the central cavity.

### SUMMARY OF THE DISCLOSURE

The invention is directed to an apparatus for providing spinal correction, comprising: an implant body configured and dimensioned for placement in an intervertebral space, said body defining a central cavity extending through said body configured to receive bone graft material and communicate with the intervertebral space for infusion of the graft material into the intervertebral space when placed therein, and a bone graft supply passage extending through said body and communicating with the central cavity; first and second extendable members mounted on said body, one each disposed on an opposite side of said central cavity, said members extendable from a first unexpanded height and to at least one expanded height; and a bone graft material supply port disposed on the implant body in communication with said bone graft supply passage, said port configured for attachment of a bone graft material supply line and having a diameter in the range of 55% to 80% of said unexpanded height; a plate with a bone engaging surface mounted on said first and second extendable members, said plate defining an opening aligned with the central cavity for passage there through of bone graft material from the central cavity.

Also disclosed is an apparatus for providing spinal correction. The apparatus includes an implant body configured and dimensioned for placement in an intervertebral space, the body defining a central cavity extending through the body configured to receive bone graft material and communicate with the intervertebral space for infusion of the graft material into the intervertebral space when placed therein, and a bone graft supply passage extending through the body and communicating with the central cavity; a bone graft material supply port disposed on the implant body in communication with the bone graft supply passage, the port configured for attachment of a bone graft material supply line; first and second extendable members mounted on the body, one each disposed on an opposite side of the central cavity, the members extendable from a first unexpanded height and to at least one expanded height; and a plate with a bone engaging surface mounted on the first and second extendable members, the plate defining an opening aligned with the central cavity for passage there through of bone graft material from the central cavity.

Yet another disclosure is directed to an apparatus for providing spinal correction. The apparatus includes an implant body configured and dimensioned for placement in an intervertebral space with a surface configured as a bone engaging surface, the body configured as a cylinder block defining first and second cylinders opening opposite the bone engaging surface and communicating with at least one hydraulic fluid passage, a central, bone graft material receiving cavity extending through the body, and a bone graft supply passage communicating with the central cavity, wherein the central cavity is configured to open to intervertebral space for infusion of the graft material into the intervertebral space when placed therein; first and second extendable pistons sealingly received in the cylinders; a top plate with an opposed bone engaging surface mounted on the first and second pistons, the top plate extendable with the pistons from a first unexpanded implant height and to at least one expanded implant height, the top plate defining an opening aligned with the central cavity for passage there through of bone graft material from the central cavity; a bone graft material supply port disposed on the implant body in communication with the bone graft supply passage, the port configured for attachment of a bone graft material supply line; a hydraulic supply port disposed on the implant body adjacent the bone graft material supply port, the hydraulic supply port communicating with the at least one hydraulic fluid passage; and an attachment port disposed on the implant body adjacent the supply port, the attachment port being configured to receive and secure an implant insertion tool.

### BRIEF DESCRIPTION OF THE DRAWINGS

For the purpose of illustrating the invention, the drawings show aspects of one or more embodiments of the invention. However, it should be understood that the present invention is not limited to the precise arrangements and instrumentalities shown in the drawings, wherein:
FIG. 1 is a representation of the vertebral column showing posterior insertion and placement of the SEC between the number 4 and 5 lumbar vertebrae according to an aspect of the invention. Whereas this diagram shows the implant anteriorly in the vertebral interspace between lumbar bones 4 and 5, the majority of lumbar fusions are performed between L5 and S1, into which implants are secured. The SEC can be used at any spinal level the surgeon deems in need of fusion;
FIG. 2 is a side view of a vertebral body showing the placement of the SEC;
FIG. 3 is a top view of a vertebral body showing placement of the SEC;
FIG. 4A is a front perspective view of the SEC in an unexpanded state;
FIG. 4B is a rear perspective view of the SEC of FIG. 4A;
FIG. 4C is a rear perspective view of the SEC of FIG. 4A showing details of the hydraulic and bone graft input ports;
FIG. 4D is a perspective view of the SEC of FIG. 4A with the wedge plate removed for clarity;
FIG. 4E is a perspective view of FIG. 4A showing the cylinders and bone graft perfusing cavity defined by the SEC body;
FIG. 4F shows another view of the wedge plate;
FIG. 4G shows details of the wedge plate and lordosis plate;
FIG. 5A is a front perspective view of the SEC in an expanded state;
FIG. 5B is a top perspective view of the SEC showing the cavity for bone graft perfusion and recesses allowing lateral movement of the wedge;
FIG. 5C is a rear perspective view of the SEC in an expanded state;
FIG. 5D is a perspective view of FIG. 5C with the SEC body removed for clarity;
FIG. 6 is a perspective view of an alternative SEC;
FIG. 7A is a perspective view of a master cylinder for hydraulic control of the SEC. A variety of alternatives are possible, most simply disposable syringes used for piston expansion;
FIG. 7B is a view of the interior of FIG. 7A;
FIG. 8 is a perspective view of an alternate master cylinder;
FIG. 9A is a perspective view of the insertion tool holding the SEC, hydraulic lines and bone graft supply line;
FIG. 9B is a close-up view of the insertion tool of FIG. 9A;
FIG. 10A shows a hydraulic line for independent control of multiple slave cylinders; and
FIG. 10B shows a close up of the fitting for the hydraulic line of FIG. 10A.
FIG. 11 is a perspective view of an SEC according to the invention.
FIG. 12 is a perspective distal view of the SEC shown in FIG. 11, with the top plate and pistons removed.
FIG. 13 shows a cross-section through line A-A in FIG. 11.
FIG. 14 is a perspective view of the SEC shown in FIG. 11 with an attached insertion tool.
FIG. 15 is a perspective view of the SEC shown in FIG. 11 with an attached bone graft material supply line.

### DETAILED DESCRIPTION

Referring to FIG. 1, vertebral segments or end plates are shown with an average 8 mm gap representing an average intervertebral space. A complete discectomy is performed prior to the insertion of the SEC 100. The intervertebral disc occupying space 102 is removed using standard techniques including rongeur, curettage, and endplate preparation to bleeding subcondral bone. The posterior longitudinal ligament is divided to permit expansion of the intervertebral space.

The intervertebral space 102 is distracted to about 10 mm using a rotating spatula (Not shown. This is a well-known device that looks like a wide screw driver that can be placed into the disc space horizontally and turned 90 degrees to separate the endplates).

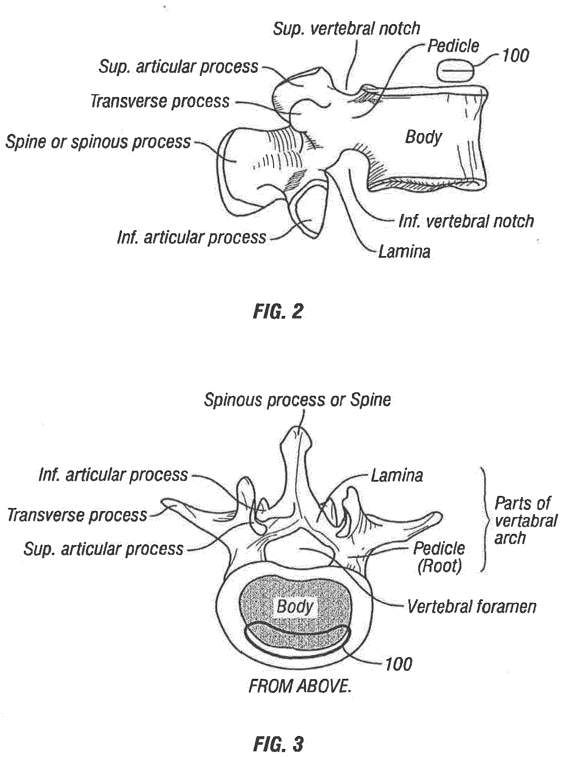

The SEC is inserted posteriorly (in the direction of arrow 102 between the no. 4 and 5 lumbar vertebrae as shown in FIG. 1 (lateral view) or into any selected intervertebral space. The SEC is reduced to small size in its unexpanded state to enable it to be inserted posteriorly through space 102 as shown in FIG. 1. Examples of dimensions of an SEC are: 12mm wide, 10mm high and 28mm long to facilitate posterior insertion and thereby minimize trauma to the patient and risk of injury to nerve roots. Once in place this exemplary SEC can expand to 16mm, or 160 percent of its unexpanded size, enabling 20 degrees or more of spinal correction medial and lateral. FIGS. 2 and 3 are a side view and top view, respectively, showing the placement of the SEC 100 on a vertebral body.

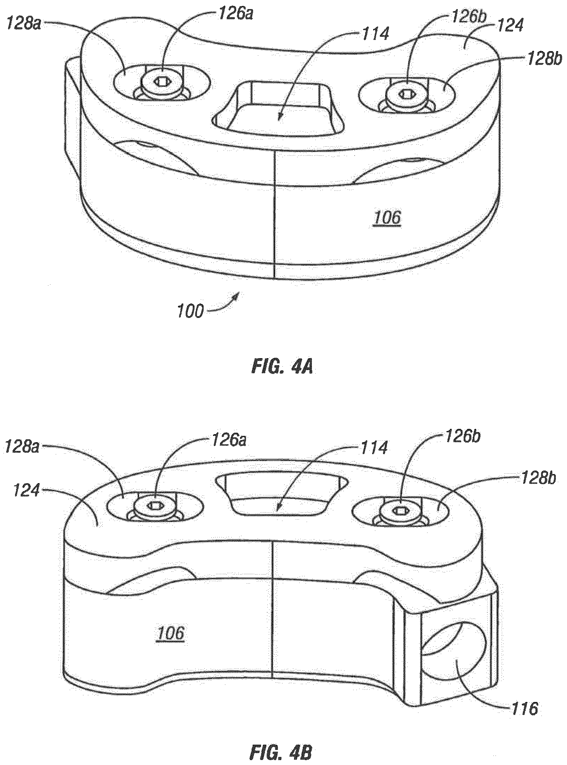

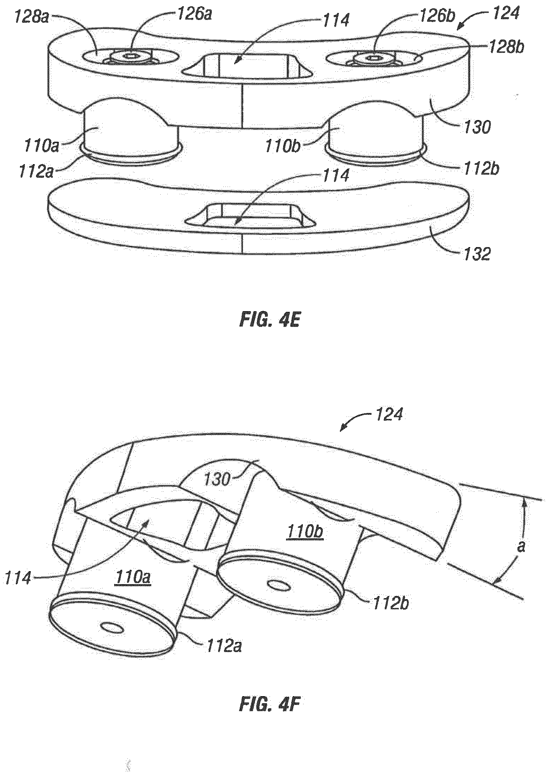

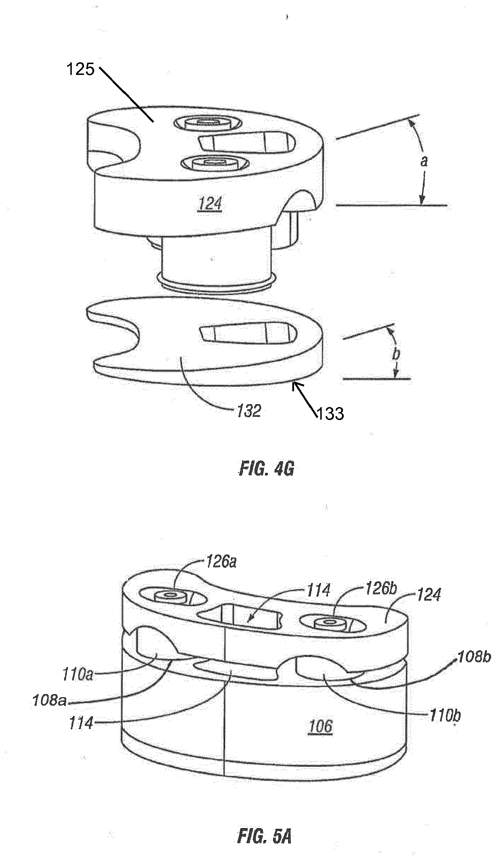

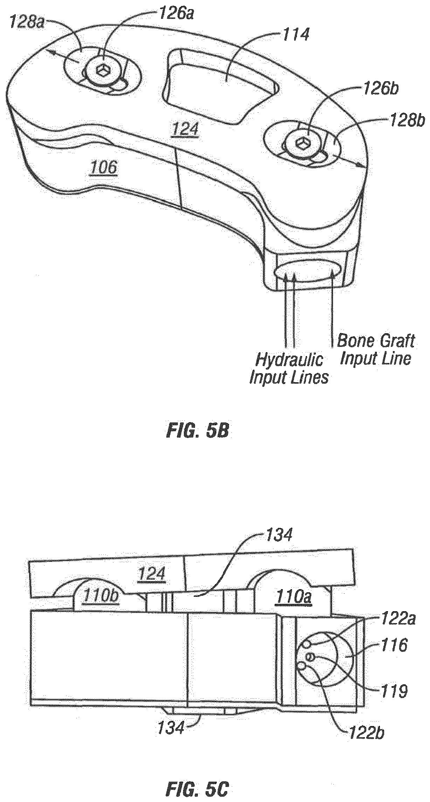

FIG. 4A shows SEC 100 from the front or anterior position with respect to the vertebral column. The SEC is shown in a closed or unexpanded position. Referring to FIGS. 4A through 4E, SEC 100 comprises a body or block 106 that defines one or more slave cylinders 108a, 108b (best seen in FIG. 5A) for corresponding pistons 110a, 110b. Pistons are provided with O-rings 112a, 112b for a tight seal with the cylinder. The pistons and cylinders cooperate to provide hydraulically extendable members disposed within the body of SEC 100 in the unexpanded state. Block 106 also defines a central cavity 114 for infusion of bone graft material into the intervertebral space when the SEC is fully expanded or during the expansion process, as will be explained.

In general, bone graft material can be any substance that facilitates bone growth and/or healing (whether naturally occurring or synthetic), such as, for example, osteoconduction (guiding the reparative growth of the natural bone), osteoinduction (encouraging undifferentiated cells to become active osteoblasts), and osteogenesis (living bone cells in the graft material contribute to bone remodeling). Osteogenesis typically only occurs with autografts.

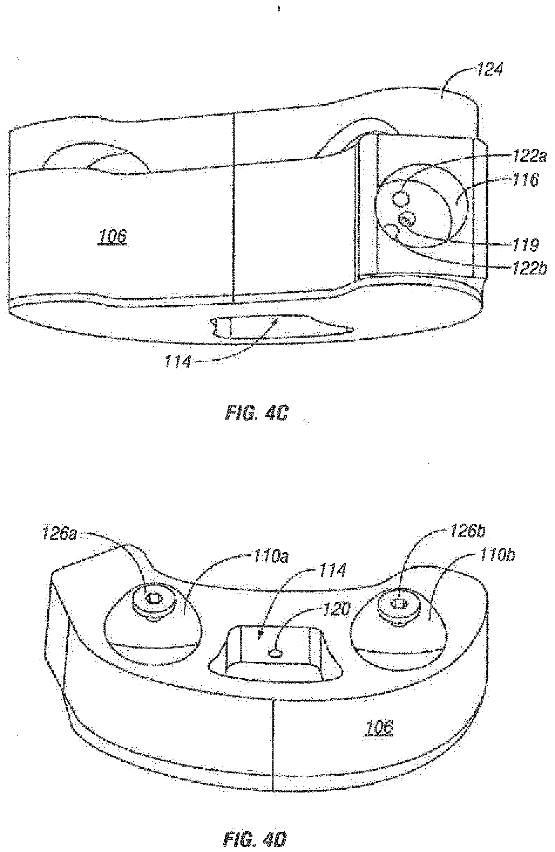

As shown in FIG. 4C, block 106 further defines a central or main input port 116 for attachment of hydraulic lines and a line for transmission of a slurry or liquid bone graft material as will be explained. The block 106 defines a bone graft infusion conduit that extends from a bone graft input port 119 located in main input port 116 to a bone graft exit port 120 (see FIG. 4D) located in central cavity 114 for infusion of bone graft material therein.

Block 106 further defines local hydraulic fluid input ports 122a, 122b (FIG. 4C) that lead to corresponding slave cylinders 108a, 108b (FIG. 5A) for driving the pistons and expanding the SEC by remote control from a master cylinder located ex vivo and with greatly increased force as compared to conventional devices.

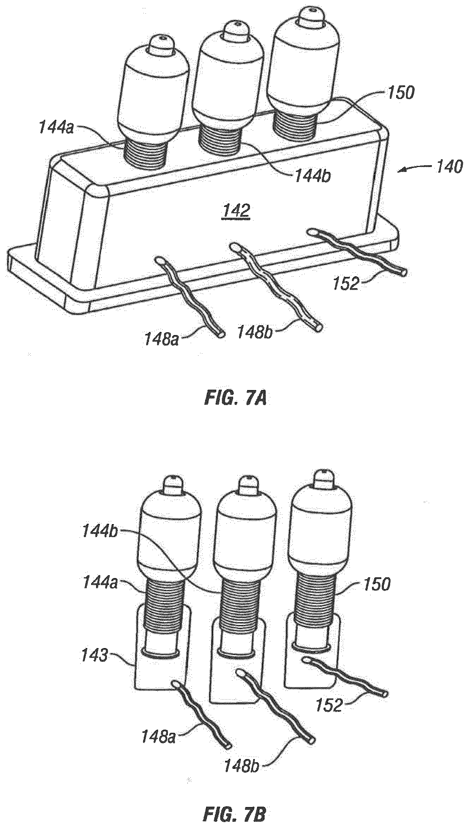

It will be appreciated that each slave piston 110a, 110b is independently controlled by a separate hydraulic line 122a, 122b connected to a master cylinder (as will be explained with reference to FIGS. 7a through 8) located away from the patient and the site of implantation, thus minimizing active intervention by surgical tools in the immediate vicinity of nerve roots. Although two slave cylinders are shown by way of example, it will be appreciated that the SEC block 106 is easily modifiable to define a multiplicity of slave cylinders, each controlled independently by a separate hydraulic line, for expanding differentially to provide a substantially infinite variety of space-sensitive adjustments for unique applications.

Referring again to FIGS. 4A through 4G, an anterior/posterior corrective plate or wedge plate 124 is movably held in captured engagement on top of pistons 110a, 110b by corresponding hold down screws 126a, and 126b. Plate 124 enables spinal correction in the anterior/posterior direction as the cylinders expand vertically. Plate 124 has a bone-engaging top surface provided with two elongated slots 128a, 128b in which the hold down screws sit. The elongated slots 128a, 128b enable ease of expansion and facilitate angles between the pistons by allowing the plate 124 to move laterally slightly as pistons differentially expand. The plate also defines cavity 114 for the infusion of bone graft material, that is co-extensive with and the same as cavity 114 defined by the SEC block. This enables perfusion of the bone graft material directly through the bone engaging surface of the wedge plate into the adjacent vertebral body.

Referring to FIGS. 4F and 4G, the anterior/posterior corrective plate 124 is provided with a downwardly-extending edge 130 for engagement with the pistons as they differentially expand, to ensure that wedge plate stays firmly in place. Plate 124 provides anterior/posterior correction in that it can be angled front to back like a wedge with a correction angle a of 0-5 degrees or more. Plate 124 also defines bone graft cavity 114 for enabling bone growth conductive or inductive agents to communicate directly with the engaged vertebral endplate.

The SEC is optionally provided with a lordosis base plate 132 that includes a bone engaging surface defining a cavity co-extensive with bone graft cavity 114 for enabling perfusion of bone graft material into the adjacent engaged vertebral body. Lordosis base plate 132 also has an anterior/posterior angle b (refer to FIG. 4G) of 0-5 degrees for correcting lordosis.

Referring to Figure 4G, top plate 124 and optional lordosis base plate 132 function as two endplates providing a corrective surface that impacts vertebral bodies for spinal correction. Top plate 124 and lordosis base plate 132 each include a bone-engaging surface 125 and 133, respectively, defining a cavity co-extensive with bone graft cavity 114 for enabling perfusion of bone graft material into the adjacent opposed vertebral body. Lordosis base plate also has anterior/posterior angle b of 0-5 degrees for correcting lordosis. Thus, the wedge plate and lordosis base plate can provide lordotic correction of 10 degrees or more.

Surgeon control over sagittal alignment is provided by differential wedge shaping of the endplates and by calculated degrees of variable piston expansion. The end plates will be constructed with 0 degrees of wedge angle anterior to posterior, or 5 degrees. Therefore, the final construct may have parallel end plates (two 0 degree endplates), 5 degrees of lordosis (one 5 degree and one 0 degree endplate), or 10 degrees of lordosis (two 5 degree implants). This implant permits unprecedented flexibility in controlling spinal alignment in the coronal and sagittal planes.

Since vertebral end plates are held together at one end by a ligament much like a clamshell, expansion of the pistons vertically against the end plates can be adjusted to create the desired anterior/posterior correction angle. Thus, the top plate 124 does not need to be configured as a wedge. Where an extreme anterior/posterior correction angle is desired, the top plate and/or base plate may be angled as a wedge with the corresponding correction angles set forth above.

Figures 5A through 5D show the SEC in its expanded state. Hydraulic fluid flows from a master cylinder (Figure 7 A) into the cylinders through separate hydraulic input lines that attach to hydraulic input ports 122a, 122b. Each hydraulic line is regulated independently thereby allowing a different quantity of material to fill each cylinder and piston cavity pushing the pistons and medial/ lateral wedge plate upward to a desired height for effecting spinal correction.

The hydraulic fluid communicating the mechanical leverage from the master cylinder to the slave cylinder or syringe and pistons advantageously is a time-controlled curable polymer such as methyl methacrylate. The viscosity and curing time can be adjusted by the formulation of an appropriate added catalyst as is well known. Such catalysts are available from LOCTITE Corp., 1001 Trout Brook Crossing, Rocky Hill, CT 06067. When the polymer cures, it hardens and locks the pistons and thus the desired amount of spinal correction determined by the physician is immovably in place.

It will be appreciated that the cylinder block 106 and pistons 110a, 110b, comprise a biocompatible, substantially incompressible material such as titanium, and preferably type 6-4 titanium alloy. Cylinder block 106 and pistons 110a, 110b completely confine the curable polymer that is acting as the hydraulic fluid for elevating the pistons. When the desired spinal correction is achieved by the expanded pistons, the curable polymer solidifies, locking the proper spinal alignment substantially invariantly in place. The confinement of the polymer by the titanium pistons and cylinder block provides the advantage of making the polymer and the desired amount of spinal alignment substantially impervious to shear and compressive forces.

For example, even if it were possible to compress the polymer it could only be compressed to the structural limit of the confining cylinder block. That is, by placing the curable polymer into the 6-4 titanium cylinder block wherein two or more cylinders are expanded, the polymer becomes essentially non-compressible especially in a lateral direction. It will be appreciated that 6-4 titanium cylinder block confining the hydraulic material provides extreme stability and resistance to lateral forces as compared to a conventional expanding implant. Further, there is no deterioration of the curable polymer over time in term of its structural integrity because it is confined in the titanium alloy body.

The use of the present 6-4 titanium cylinder block configuration can withstand compressive forces in excess of 12,000 Newtons or approximately 3000 pounds of compressive force on the vertebrae. This is not possible in a conventional expanding structure wherein the expanding polymer is not confined by an essentially incompressible titanium body.

Injectable bone graft material 134 may be provided along a separate bone graft input line to bone graft input port 119 for infusion into cavity 114 through bone graft exit port 120.

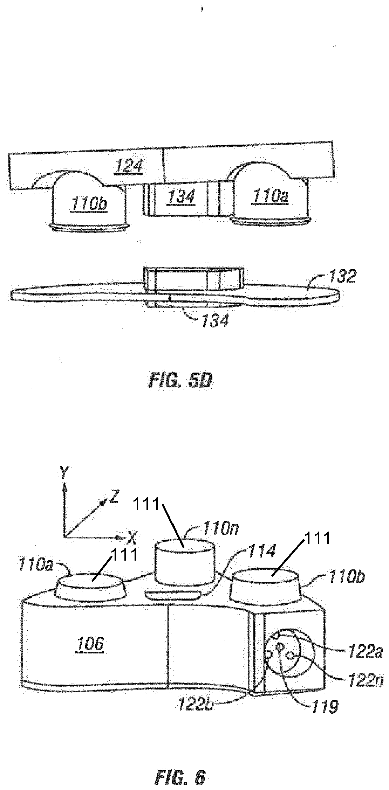

The bone graft input line is controlled at the master cylinder or from a separate source to enable a pressure-induced infusion of bone graft material 134 through cavity of the bone engaging surfaces of the SEC into adjacent vertebral bone. Thus, the bone graft material fills, under pressure, the post-expansion space between adjacent vertebral bodies. This achieves substantially complete perfusion of osteo-inductive and/or osteo-conductive bone graft material in the post expansion space between the vertebral bodies resulting in enhanced fusion (refer to FIGS. 5C, 5D).

Referring to FIG. 6, an alternate SEC comprises multiple slave cylinders and corresponding pistons 110a, 110b, 110n are provided in SEC body 106. Each of the multiple slave cylinders and pistons 110a, 110b, 110n is provided with a separate, associated hydraulic line 122a, 122b, 122n that communicates independently with a corresponding one of a plurality of cylinders in the master cylinder for independently controlled expansion of the slave cylinders at multiple elevations in three dimensions (X, Y and Z axes).

At the master cylinder, multiple threaded cylinders (or disposable syringes) and pistons are provided, each communicating independently through a separate hydraulic line 122a, 122b, 122n with a corresponding one of the slave cylinders and pistons 110a, 110b, 110n in the SEC.

The bone engaging surfaces of the multiple pistons 110a, 110b, 110n provide the corrective surface of the SEC. Thus, by appropriate adjustment of the pistons in the master cylinder, or depending on fluid installed via separate syringes, the surgeon can independently control expansion of the slave pistons in the SEC to achieve multiple elevations in three dimensions for specialized corrective applications. A top or wedge plate is not necessary.

The bone engaging surface 111 of the slave pistons 110a, 110b, 110n in the SEC may be provided with a specialized coating for bone ingrowth such as hydroxyapetite. Alternatively, the bone-engaging surface 111 of the SEC pistons may be corrugated, or otherwise provided with a series of bone engaging projections or cavities to enhance fusion.

As previously explained, the hydraulic fluid communicating the mechanical leverage from the master cylinder to the SEC slave cylinders and pistons 110a, 110b, 110n is a time-controlled curable polymer such as methyl methacrylate that locks the SEC immovably in place after curing, at the desired three dimensional expansion.

As set forth above, injectable bone graft material is provided along a separate bone graft input line to bone graft input port 119 for infusion into cavity 114 and into the inter body space between the SEC and adjacent bone.

The surgeon by adjustment of the master cylinder is able to provide remotely a controlled angle of the SEC corrective surface to the medial/lateral (X axis) and in the anterior, posterior direction (Z axis). The surgeon also can adjust the SEC in the vertical plane moving superiorly/inferiorly (Y axis) from the master cylinder or power/flow source to control implant height. Thus, three-dimensional control is achieved remotely through a hydraulic line with minimal trauma to a patient. This obviates the need to manually manipulate the SEC implant at the site of intervention to achieve desired angles of expansion. Such conventional manual manipulation with surgical tools into the intervention site can require further distracting of nerve roots and cause potential serious trauma to a patient.

Referring to FIGS. 7A and 7B, a master cylinder 140 located remotely from the patient, provides controlled manipulation and adjustment of the SEC in three dimensions through independent hydraulic control of slave cylinders 110a, 110b in the SEC. Master cylinder 140 comprises a cylinder block 142, defining two or more threaded cylinders 143. Corresponding screw down threaded pistons are rotated downward into the threaded cylinders thereby applying force to a hydraulic fluid in corresponding hydraulic control lines that communicate independently with and activate corresponding slave cylinders 110a, 110b in the SEC with mechanical leverage. The rotational force for applying the mechanical leverage at the slave cylinders is controlled by thread pitch of the threaded pistons in the master cylinder, or alternatively controlled by use of syringes, one acting as a master cylinder for each piston or slave cylinder to modulate piston elevation.

In FIG. 7B threaded pistons 144a, 144b are provided in hydraulic cylinders communicating through hydraulic lines 148a, 148b that are coupled to hydraulic input ports 116a, 116b for independent hydraulic control of slave cylinders 110a, 110b as previously explained.

Another threaded cylinder and piston assembly 150 is supplied with a quantity of bone graft material in slurry or liquid form and operates in the same way to provide the bone graft material under pressure to the SEC bone graft input port 119 through bone graft supply line 152. Thus, bone graft material is forced under pressure from the master cylinder through cavity 114 and into the intervertebral space.

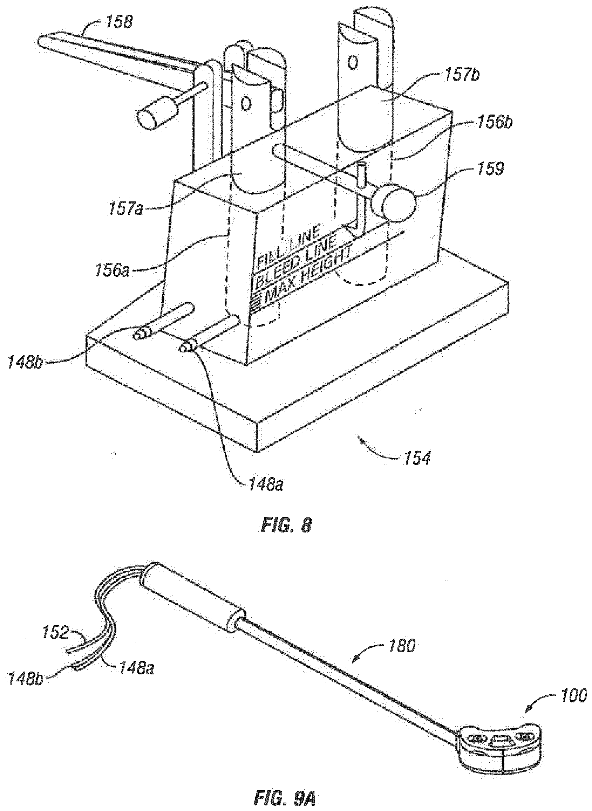

Referring to FIG. 8, a master cylinder is provided for individual hydraulic control of each slave piston in the SEC implant. A master cylinder 154 is provided with two or more cylinders 156a, 156b, and associated pistons 157a, 157b. A lever 158 controlled by the surgeon is attached to each piston. Hydraulic fluid feeds through lines 148a 148b into the inserted SEC implant. The lever creates a ratio of 1 pound to 10 pounds of pressure inside the slave cylinders in the SEC and thus against vertebral end plates. Mechanically this provides a 10:1 advantage in lift force for the surgeon. The surgeon's required force application is multiplied via the lever and hydraulic system to create a controlled expansion of the SEC against the end plates as previously described to create any desired spine vertebral correctional effect in three dimensions.

If the surgeon uses one pound of force on the lever, the piston exerts 10 pounds of force. The piston in the master cylinder displaces the hydraulic fluid through hydraulic lines 148a, 148b. The hydraulic lines are flexible conduit no more than 3 mm in diameter. Thin hydraulic lines are desirable to increase mechanical advantage at the slave cylinders in the SEC. If one pound of pressure (0,45 kg) is exerted on the handle, the corresponding piston in the SEC would have 10 pounds (4,54 kg) of lifting force. If each slave piston inside the SEC implant has 200 pounds (90,71 kg) of lifting force, the required amount of pressure applied by the surgeon to the master piston cylinder is 20 pounds (9,07 kg) or one tenth the amount, consistent with the predetermined mechanical advantage.

In usual cases, where the surgeon has a patient in a partially distracted anatomic, anesthetized and relaxed position under anesthesia, 30 pounds (13,61 kg) of force may be required for implant expansion upon the vertebral bone endplates. The surgeon in that case would need to apply only 3 pounds (1,36kg) of pressure to lever 158. Different ratios may be introduced to optimize distraction force while minimizing injection pressures.

The pressure application process is guided by normal surgical principles, by visual checkpoints, and by a safety gauge that illustrates the amount of expansion that has been exerted in direct correlation with the implant expansion process. The gauge indicates the height of the slave pistons and thus the vertical and angular expansion of the SEC. This translates to an ability to clarify the percentage of lateral expansion. That is, if the surgeon chooses to create an angle, he expands the right slave cylinder, for example, 14 mm and left slave cylinder 12 mm.

The master cylinder 154 preferably comprises transparent plastic to enable visual indication of the height of the hydraulic fluid therein, or a translucent plastic syringe to facilitate exact measured infusion of the slave cylinder implant expanding pistons. A knob 159 for setting gauge height is provided in each cylinder. An indicator attached to the knob registers the cylinder height with respect to a fill line, bleed line or maximum height line. The master cylinder and slave cylinders are filled with hydraulic fluid. Air is removed by bleeding the cylinders in a well-known manner. The knob indicator is registered to the bleed line. A series of incremental marks are provided between the bleed line and the maximum height line to show the surgeon the exact height of the slave cylinder in response to the surgeon's control inputs to the master cylinder.

It will be appreciated that the master and slave hydraulic system interaction can have many equivalent variations. For example, the master cylinder function of master cylinder 154 also can be provided by one or more syringes. Each syringe acts as a master cylinder and is coupled independently with a corresponding slave cylinder through a thin hydraulic line for independent activation as previously described. A single syringe acting as a master cylinder also may be selectively coupled with one or more slave cylinders for independent activation of the slave cylinders. As is well known, series of gradations are provided along the length of the syringe that are calibrated to enable the surgeon to effect a precise elevation of a selected piston at the corresponding slave cylinder in the implant.

As previously explained, the SEC implant also expands vertically the intervertebral space from 10 mm to 16 mm or more. Additionally, by changing the diameter of the piston inside the master cylinder, the force exerted into the slave cylinder could be multiplied many fold so as to create major force differentials. The foregoing features provide the surgeon with an ability to establish a spinal correction system that is a function of the needed change to correct a deformity, so as to produce normal alignment.

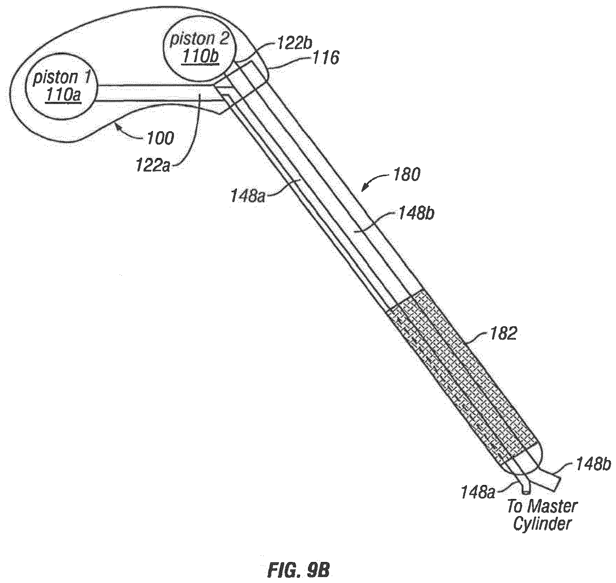

Referring to FIG. 9A, it will be appreciated that hydraulic control lines 148a and 148b and bone graft supply line 152 are characterized by a minimal size and are provided in the interior of a very narrow insertion tool 180 (Figures 9A and 9B). The insertion tool 180 is small enough to insert the SEC 100 posteriorly into the narrow insertion opening without risk of serious trauma to the patient. An enlarged view of the insertion tool 180 (simplified for clarity) is shown in FIG. 9B. The insertion tool 180 includes a handle 182 and hollow interior for housing hydraulic control lines and a bone graft supply line (not shown for clarity). The hydraulic control lines and bone graft supply line connect through a proximal end of the insertion tool to the master cylinder. A distal or insertion end of the tool holds the SEC 100. In a preferred mode, the insertion end of the insertion tool conformably fits in the SEC hydraulic input port 116. Hydraulic control lines and the bone graft supply line are connected to the hydraulic input ports 122a, 122b and bone graft supply input port respectively, prior to surgery.

The bone graft supply and hydraulic control lines are safely retracted after the SEC is positioned. The hydraulic lines can be released by cutting after the operation since the hydraulic fluid hardens in place.

When the SEC is locked in position by the surgeon, the insertion tool and hydraulic tubes are removed and the curable polymer remains in the SEC slave cylinders.

The hydraulic fluid controlling the movement of the SEC may be a time-controlled curable polymer that hardens after a pre-determined time period, locking the SEC insert immovably in a desired expanded position. The hydraulic fluid is preferably methylmethacrylate or other similar inexpensive polymer, with a time-controlled curing rate. Time-controlled curable polymers typically comprise a catalyst and a polymer. The catalyst can be formulated in a well-known manner to determine the time at which the polymer solidifies. Such time-controlled curable polymers are commercially available from several manufacturers such as LOCTITE Corp., Henkel-Loctite, 1001 Trout Brook Crossing, Rocky Hill, CT 06067.

As is well understood by one skilled in the art, any equivalent curable polymer that has a first flowable state for conveying hydraulic force, and that transitions to a second solid state upon curing may be employed. In the first state, the curable polymer transfers the application of force hydraulically from the master cylinder to the slave cylinders, such that corrective action is achieved by elevating the slave pistons. The curable polymer transitions to a second solid state upon curing such that the corrective elevation of the slave pistons is locked in place. Such an equivalent curable polymer is a polymer that is cured through the application of either visible or ultraviolet light or other radiation source which activates the polymer to transition to a solid state. Another methyl methacrylate liquid polymer when combined with powder becomes a viscous fluid as soon as the powder and liquid are blended; it is initially thin and free flowing. Gradually, in minutes, it begins to thicken, transforming state through paste and puddy to cement-like solid once inside the pistons, thus fixing the SEC at a precise correction amount in its expanded position.

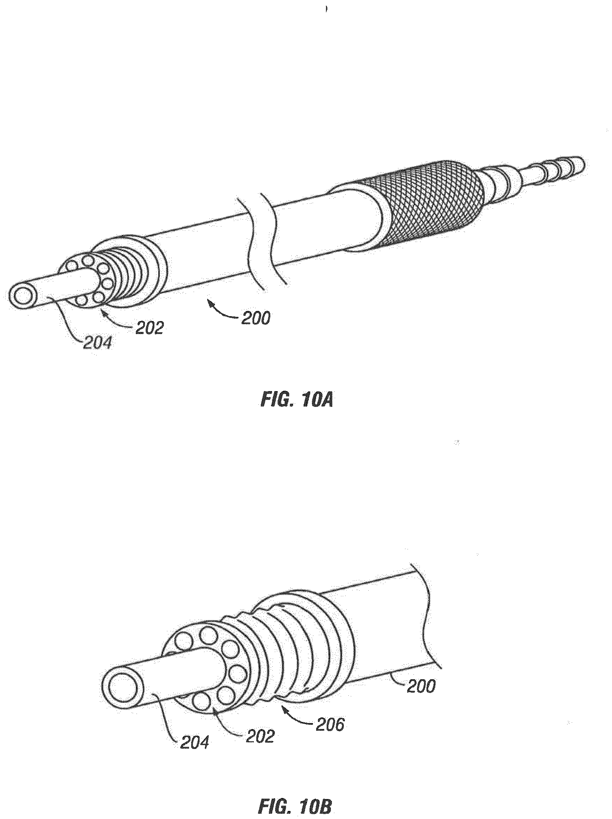

An example of such a light curable polymer is UV10LC-12 made by MASTER BOND Inc., of Hackensack, N.J. Such polymers are characterized by a fast cure time upon exposure to a visible or a UV light source. Depending upon the intensity of the light source, cure times range from a few seconds to less than a minute. As is well understood by one skilled in the art, an extremely thin fiber optic line may be incorporated as an additional line along with the multiple hydraulic lines shown in FIGS. 10A and 10B for conveying light from a light source directly to the polymer in the slave cylinders to effect curing.

Alternatively, a curable polymer may be activated by a radiation source such as low level electron beam radiation to cure or initiate curing. An electron beam advantageously can penetrate through material that is opaque to UV light and can be applied directly to lock the pistons in their elevated or corrective position.

It will be appreciated that the amount of applied stress required to cause failure of the corrective implant is substantial due to the confinement of the cured polymer completely within the body of the implant, that is, the cylinder block that is comprised of 6-4 titanium. This is particularly advantageous since the confinement within the titanium body enables the corrective position of the implant to withstand compressive forces up to the structural failure limit of the titanium body; that is, to withstand compressive forces in a range of from 8000 up to 12,000 Newtons.

Referring to FIGS. 10A and 10B, a hydraulic line 200 is provided for remote hydraulic control of a plurality of slave cylinders of the SEC from a master cylinder. Hydraulic line 200 comprises a plurality of individual hydraulic lines 202 disposed about a central axis. Each hydraulic line 202 provides independent activation of a separate slave cylinder from a master cylinder as previously explained. A bone graft supply line 204 is provided along the central axis of line 200. Individual hydraulic lines 202 can be aligned and connected with corresponding slave cylinder input ports prior to insertion of the SEC for providing independent hydraulic control to each of the slave cylinders. A threaded end 206 can be inserted into a similarly threaded central input port 116 of the SEC to prevent pull out.

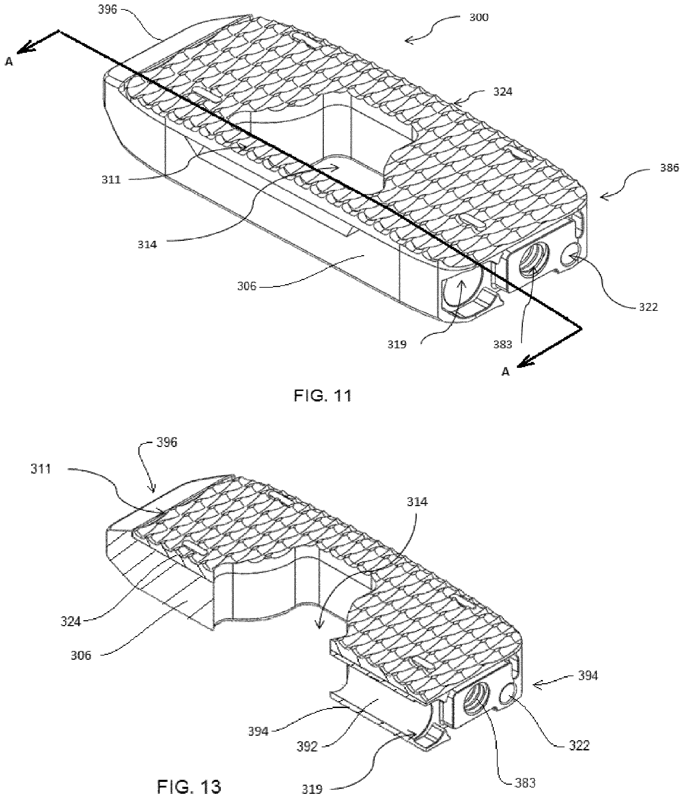

According to the present invention, as illustrated in FIGS. 11-15, SEC 300 includes a block or body 306 defining cylinders 308 for receiving pistons cooperating with a top plate 324 substantially as previously described. The body 306 of SEC 300 also defines a central cavity 314 to receive bone graft material for communication with the intervertebral space when implanted. Top plate 324 also provides a central opening aligned with central cavity 314 to facilitate such communication. As shown, for example in FIG. 11, top plate 324 may be provided with a textured bone engagement surface 311 on the superior surface thereof and the central opening may be shaped to match the shape of central cavity 314. The textured surface is configured to provide for greater security and fixation between the vertebral body and SEC 300, and is not limited to the pattern shown, but may be of any appropriate configuration for enhancing fixation as will be appreciated by persons of ordinary skill in the art.

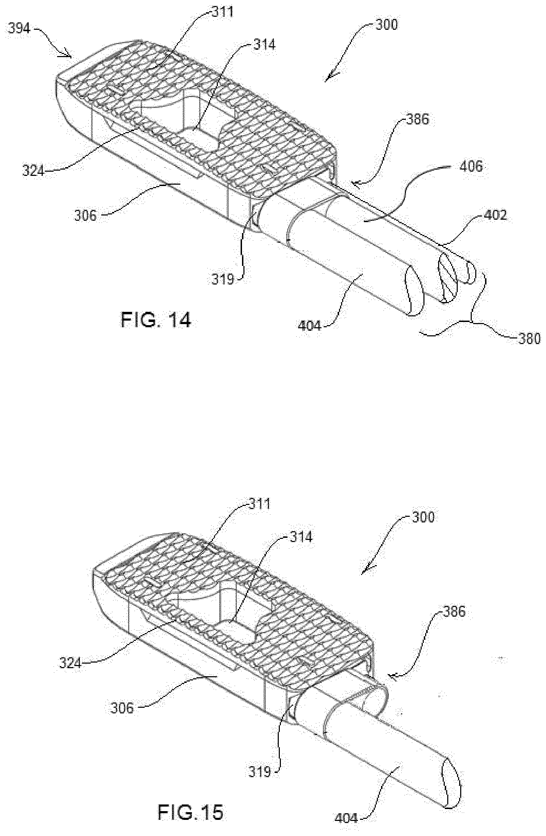

In this exemplary embodiment, SEC 300 includes a graft infusion port 319 in communication with the central graft cavity 314, which may be positioned laterally on a proximal face 386 of body 306 as shown in FIG. 11. Graft infusion port 319 may be located laterally of an attachment port 383, which is where the insertion tool 380 is connected to the SEC 300 via a threaded connector and rotary actuator 406 (see FIG. 14). Hydraulic line port 322, communicating with passages leading to cylinders 308, may be located laterally opposite attachment port 383 on proximal face 386 to receive hydraulic supply line 402 for actuation of the pistons. Distal face 396, opposite the attachment port, may present narrowed leading edge to facilitate insertion and placement of SEC 300 between adjacent vertebral bodies.

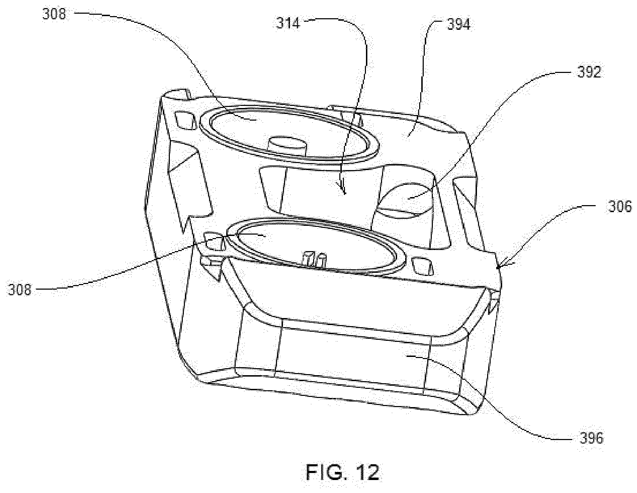

As shown, for example, in FIGS. 12 and 13, the graft infusion port 319 communicates with the central cavity 314 through passage 392, which traverses the proximal wall 394 of block 306. Graft slurry or other bone growth promoting material infused through the graft port 319 flows directly into the central cavity 314 from passage 392. The relatively large diameter of port 319 and passage 392 allow for unobstructed flow of material into the central cavity. Depending on the size of the implant and the amount of height to be achieved through extension of the pistons, this can be particularly valuable because the central cavity 314 enlarges as the SEC 300 expands. A free flow of bone graft material with sufficient volume is required to fill the enlarged volume of central cavity 314 after expansion. For example, in an implant with a width of about 18mm, a length of about 50mm, and a height of about 8mm, which is expanded after placement to a height of about 12mm, passage 392 may have an internal diameter of about 6 mm. In general, to provide an unobstructed flow of bone graft material, particularly in slurry form, according to the invention passage 392 (and port 319) is sized such that the ratio of the diameter of passage to the unexpanded implant height is at least about 55% and more preferably at least about 60%. The ratio of passage diameter to unexpanded implant height is in the range of about 55-80% or more specifically about 60-75%.

As previously mentioned, with the graft infusion port 319 located lateral of the attachment port 383, the bone graft supply line 404 of insertion tool 380 can also be located lateral to the hydraulic lines 402 as shown in FIG. 14. However, this parallel, lateral arrangement may make insertion tool 380 wider, possibly creating difficulty in passing sensitive neural structures in some anatomies. Where the lateral width of the insertion tool is of concern, such concern may be addressed by providing the bone graft supply line 404 separate from an insertion tool including only the rotary actuator 406 and hydraulic supply line 402, such that bone graft supply line 404 may be placed separately and only after the SEC 300 is implanted and expanded, and the insertion tool removed, as shown in FIG. 15. In this case, a collar may be provided that also attaches in attachment port 319 to provide additional security for the bone graft supply line attachment.

In summary, remote hydraulic control of a spinal implant is particularly advantageous in a posterior insertion procedure because there is no anatomic room for mechanical linkage or tooling in the proximity of the adjacent spinal cord and neurovascular complex. The hydraulic control provided by the present invention provides significant mechanical leverage and thus increased force to an extent that has not previously been possible. Further, such hydraulic force is selective in both direction and magnitude of its application.

It is now possible to expand fenestrated endplates to support the anterior spinal column. This will create immediate and reliable firm fixation that will lead to immediate stabilization of the functional spinal motion segment, and immediate correction of complex interbody deformities in the sagittal and coronal plane.

The SEC provides advantages over currently existing technology that include correction of coronal plane deformity; introduction of interbody lordosis and early stabilization of the interbody space with rigidity that is greater than present spacer devices. This early stability may improve postoperative pain, preclude the need for posterior implants including pedicle screws, and improve the rate of successful arthrodesis. Importantly, the SEC provides improvement of space available for the neural elements while improving lordosis. Traditional implants are limited to spacer effects, as passive fillers of the intervertebral disc locations awaiting eventual fusion if and when bone graft in and around the implant fuses. By expanding and morphing into the calculated shape which physiologically corrects spine angulation, the SEC immediately fixes the spine in its proper, painless, functional position. As infused osteoinductive/osteoconductive bone graft materials heal, the patient becomes well and the implant becomes inert and quiescent, embedded in bone, and no longer needed.

While the invention has been described in connection with what are presently considered to be the most practical and preferred embodiments, it is to be understood that the invention is not limited to the disclosed exemplary embodiments and alternatives as set forth above, but on the contrary is intended to cover various modifications and equivalent arrangements included within the scope of the following claims.

For example, equivalent expansion surfaces can be provided for stabilizing the expanding SEC against the bone. Other compositions of additives may be used for the hydraulic fluid that achieves remote controlled expansion of the SEC in three dimensions. Similarly, various types of biogenic fluid material for enhancing bone growth may be injected through one or more lines to the SEC and different exit apertures may be provided to apply bone graft material to fill the intervertebral space, without departing from the scope of the invention.

The implant itself can be made of, for example, such materials as titanium, 64 titanium, or an alloy thereof, 316 or 321 stainless steel, biodegradeable and biologically active materials, e.g. stem cells, and polymers, such as semi-crystalline, high purity polymers comprised of repeating monomers of two ether groups and a key tone group, e.g. polyaryetheretherketone (PEEK) TM, or teflon.

Finally, the implant may provide two or more pistons that are operated concurrently to provide coordinated medial/lateral adjustment of a patient's spine for scoliosis, with anterior/posterior adjustment of the patient's spine to create natural lordosis, with relative anterior expansion greater than posterior expansion.

Therefore, persons of ordinary skill in this field are to understand that all such equivalent processes, arrangements and modifications are to be included within the scope of the following claims.

Exemplary embodiments have been disclosed above and illustrated in the accompanying drawings. It will be understood by those skilled in the art that various changes, omissions and additions may be made to that which is specifically disclosed herein without departing from the scope of the present invention.

## Claims

1. An apparatus (300) for providing spinal correction, comprising:
an implant body (306) configured and dimensioned for placement in an intervertebral space (102), said body (306) defining a central cavity (314) extending through said body (306) configured to receive bone graft material and communicate with the intervertebral space (102) for infusion of the graft material into the intervertebral space (102) when placed therein, and a bone graft supply passage (392) extending through said body (306) and communicating with the central cavity (314); first and second extendable members (308) mounted on said body (306), one each disposed on an opposite side of said central cavity (314), said members (308) extendable from a first unexpanded height and to at least one expanded height; and
a bone graft material supply port (319) disposed on the implant body (306) in communication with said bone graft supply passage (392), said port (319) configured for attachment of a bone graft material supply line (404) and having a diameter in the range of 55% to 80% of said unexpanded height;
a plate (324) with a bone engaging surface (311) mounted on said first and second extendable members (308), said plate (324) defining an opening aligned with the central cavity (314) for passage there through of bone graft material from the central cavity (314).

2. The apparatus of claim 1, wherein the implant body defines a bone engaging surface opposite the bone engaging surface of said plate and the central cavity is defined between said surfaces and opens through said surfaces for communication with the intervertebral space.

3. The apparatus of claim 1, wherein the diameter of the bone graft supply passage is in the range of 60% to 75% of said unexpanded height.

4. The apparatus of claim 1, wherein said first and second extendable members comprise:
first and second cylinders defined in said implant body;
first and second pistons received, respectively in said first and second cylinders; and
said implant body defines a cylinder block containing said cylinders.

5. The apparatus of claim 4, wherein said pistons are hydraulically extendable with an incompressible fluid and said implant body further defines a hydraulic supply port and at least one hydraulic fluid passage communicating between said hydraulic supply port and said cylinders.

6. The apparatus of claim 5, wherein the said pistons are lockable in an extended position at a desired spinal correction.

7. The apparatus of claim 6, wherein the incompressible fluid comprises a curable polymer having a fluid state for providing hydraulic force to elevate said pistons to a position that provides the desired spinal correction, and said polymer cures to a solid state to lock said pistons at said desired correction.

8. The apparatus of claim 5, wherein:
the implant body further defines an attachment port adjacent said bone graft material supply port; and
said apparatus further comprises an insertion tool releasably engageable with said attachment port for positioning the implant.

9. The apparatus of claim 8, wherein:
the cylinders defined in the implant body comprise slave cylinders;
said apparatus further comprises at least one remote master cylinder for providing fluid under pressure to said slave cylinders; and
said insertion tool is engageable with the hydraulic supply port and includes a passage for delivering hydraulic fluid under pressure from said master cylinder.

10. The apparatus of claim 9, wherein said insertion tool is engageable with the bone graft material supply port and includes a passage for delivering bone graft material to said port from a remote supply of said material.

11. The apparatus of claim 10, wherein said ports are disposed on a proximal face of the implant body, laterally spaced apart.

12. The apparatus of claim 11, wherein a distal face of the implant body comprises a narrowed leading edge to facilitate insertion and placement of the implant body in the intervertebral space.

## Patentansprüche

1. Vorrichtung (300) zum Bereitstellen einer Wirbelsäulenkorrektur, Folgendes umfassend:
einen Implantatkörper (306), konfiguriert und dimensioniert zum Platzieren in einem Intervertebralraum (102), wobei der Körper (306) einen mittleren Hohlraum (314) definiert, der sich durch den Körper (306) hindurch erstreckt, um Knochentransplantatmaterial aufzunehmen und mit dem Intervertebralraum (102) zu kommunizieren, um das Transplantatmaterial in den Intervertebralraum (102) einzuführen, wenn darin platziert, und einen Knochentransplantatversorgungsdurchlass (392), der sich durch den Körper (306) erstreckt und mit dem mittleren Hohlraum (314) kommuniziert;
ein erstes und ein zweites ausfahrbares Element (308), befestigt an dem Körper (306), jeweils eines davon angeordnet an einer gegenüberliegenden Seite des mittleren Hohlraums (314), wobei die Elemente (308) von einer ersten nicht ausgefahrenen Höhe und bis zu wenigstens einer ausgefahrenen Höhe ausfahrbar sind; und
einen Knochentransplantatmaterialversorgungsanschluss (319), angeordnet an dem Implantatkörper (306) in Kommunikation mit dem Knochentransplantatversorgungsdurchlass (392), wobei der Anschluss (319) konfiguriert ist zum Anbauen einer Knochentransplantatmaterialversorgungsleitung (404) und mit einem Durchmesser im Bereich von 55 % bis 80 % der nicht ausgefahrenen Höhe;
eine Platte (324) mit einer Knochenansatzoberfläche (311), befestigt an dem ersten und dem zweiten ausfahrbaren Element (308), wobei die Platte (324) eine an dem mittleren Hohlraum (314) ausgerichtete Öffnung definiert, damit Knochentransplantatmaterial von dem mittleren Hohlraum (314) aus dadurch passieren kann.

2. Vorrichtung nach Anspruch 1, wobei der Implantatkörper eine Knochenansatzoberfläche gegenüber von der Knochenansatzoberfläche der Platte definiert und wobei der mittlere Hohlraum zwischen den Oberflächen definiert ist und sich für Kommunikation mit dem Intervertebralraum durch die Oberflächen hindurch öffnet.

3. Vorrichtung nach Anspruch 1, wobei der Durchmesser des Knochentransplantatversorgungsdurchlasses im Bereich von 60 % bis 75 % der nicht ausgefahrenen Höhe liegt.

4. Vorrichtung nach Anspruch 1, wobei das erste und das zweite ausfahrbare Element Folgendes umfassen:
einen ersten und einen zweiten Zylinder, definiert in dem Implantatkörper;
einen ersten und einen zweiten Kolben, aufgenommen in dem ersten beziehungsweise dem zweiten Zylinder; und
wobei der Implantatkörper einen Zylinderblock definiert, der die Zylinder enthält.

5. Vorrichtung nach Anspruch 4, wobei die Kolben mit einem nicht komprimierbaren Fluid hydraulisch ausfahrbar sind und wobei der Implantatkörper ferner einen hydraulischen Versorgungsanschluss und wenigstens einen Hydraulikfluiddurchlass, der zwischen dem hydraulischen Versorgungsanschluss und den Zylindern kommuniziert, definiert.

6. Vorrichtung nach Anspruch 5, wobei die Kolben in einer ausgefahrenen Position bei einer gewünschten Wirbelsäulenkorrektur verriegelbar sind.

7. Vorrichtung nach Anspruch 6, wobei das nicht komprimierbare Fluid ein aushärtbares Polymer umfasst, das einen Fluidzustand aufweist, um Hydraulikkraft bereitzustellen, um die Kolben auf eine Position anzuheben, die die erwünschte Wirbelsäulenkorrektur bereitstellt, und wobei das Polymer in einen festen Zustand aushärtet, um die Kolben in der erwünschten Position zu verriegeln.

8. Vorrichtung nach Anspruch 5, wobei:
der Implantatkörper ferner einen Anbauanschluss neben dem Knochentransplantatmaterialversorgungsanschluss bereitstellt; und
die Vorrichtung ferner ein Einführwerkzeug umfasst, das lösbar mit dem Anbauanschluss in Eingriff zu bringen ist, um das Implantat zu positionieren.

9. Vorrichtung nach Anspruch 8, wobei:
die in dem Implantatkörper definierten Zylinder untergeordnete Zylinder umfassen;
die Vorrichtung ferner wenigstens einen entfernt angeordneten Hauptzylinder umfasst, um Fluid unter Druck an die untergeordneten Zylinder bereitzustellen; und
das Einführwerkzeug mit dem hydraulischen Versorgungsanschluss in Eingriff zu bringen ist und einen Durchlass zum Bereitstellen von Hydraulikfluid unter Druck von dem Hauptzylinder enthält.

10. Vorrichtung nach Anspruch 9, wobei das Einführwerkzeug mit dem Knochentransplantatmaterialversorgungsanschluss in Eingriff zu bringen ist und einen Durchlass enthält, um Knochentransplantatmaterial von einer entfernt angeordneten Versorgung mit dem Material an den Anschluss zu liefern.

11. Vorrichtung nach Anspruch 10, wobei die Anschlüsse, lateral voneinander beabstandet, an einer proximalen Fläche des Implantatkörpers angeordnet sind.

12. Vorrichtung nach Anspruch 11, wobei eine distale Fläche des Implantatkörpers eine verjüngte Führungskante umfasst, um ein Einführen und Platzieren des Implantatkörpers in dem Intervertebralraum zu ermöglichen.

## Revendications

1. Appareil (300) pour fournir une correction rachidienne, comprenant :
un corps d'implant (306) configuré et dimensionné pour être placé dans un espace intervertébral (102), ledit corps (306) définissant une cavité centrale (314) s'étendant à travers ledit corps (306) configurée pour recevoir un matériau de greffon osseux et communiquer avec l'espace intervertébral (102) pour une infusion du matériau de greffon dans l'espace intervertébral (102) lorsqu'il y est placé, et un passage d'alimentation en greffon osseux (392) s'étendant à travers ledit corps (306) et communiquant avec la cavité centrale (314) ;
des premier et second organes extensibles (308) montés sur ledit corps (306), chacun étant disposé sur un côté opposé de ladite cavité centrale (314), lesdits organes (308) étant extensibles depuis une première hauteur non agrandie et jusqu'à au moins une hauteur agrandie ; et
un orifice d'alimentation en matériau de greffon osseux (319) disposé sur le corps d'implant (306) en communication avec ledit passage d'alimentation en greffon osseux (392), ledit orifice (319) étant configuré pour fixer une ligne d'alimentation en matériau de greffon osseux (404) et ayant un diamètre dans la plage de 55 % à 80 % de ladite hauteur non agrandie ;
une plaque (324) avec une surface de coopération osseuse (311) montée sur lesdits premier et second organes extensibles (308), ladite plaque (324) définissant une ouverture alignée avec la cavité centrale (314) pour le passage à travers celle-ci de matériau de greffon osseux depuis la cavité centrale (314).

2. Appareil selon la revendication 1, dans lequel le corps d'implant définit une surface de coopération osseuse opposée à la surface de coopération osseuse de ladite plaque et la cavité centrale est définie entre lesdites surfaces et s'ouvre à travers lesdites surfaces pour communiquer avec l'espace intervertébral.

3. Appareil selon la revendication 1, dans lequel le diamètre du passage d'alimentation en greffon osseux est dans la plage de 60 % à 75 % de ladite hauteur non agrandie.

4. Appareil selon la revendication 1, dans lequel lesdits premier et second organes extensibles comprennent :
des premier et second cylindres définis dans ledit corps d'implant ;
des premier et second pistons reçus, respectivement dans lesdits premier et second cylindres ; et
ledit corps d'implant définit un bloc-cylindres contenant lesdits cylindres.

5. Appareil selon la revendication 4, dans lequel lesdits pistons sont extensibles hydrauliquement avec un fluide incompressible et ledit corps d'implant définit en outre un orifice d'alimentation hydraulique et au moins un passage de fluide hydraulique communiquant entre ledit orifice d'alimentation hydraulique et lesdits cylindres.

6. Appareil selon la revendication 5, dans lequel lesdits pistons sont verrouillables dans une position étendue au niveau d'une correction rachidienne souhaitée.

7. Appareil selon la revendication 6, dans lequel le fluide incompressible comprend un polymère durcissable ayant un état fluide pour fournir une force hydraulique afin d'élever lesdits pistons jusqu'à une position qui fournit la correction rachidienne souhaitée, et ledit polymère se durcit en un état solide pour verrouiller lesdits pistons au niveau de ladite correction souhaitée.

8. Appareil selon la revendication 5, dans lequel :
le corps d'implant définit en outre un orifice de fixation adjacent audit orifice d'alimentation en matériau de greffon osseux ; et
ledit appareil comprend en outre un outil d'insertion pouvant coopérer de façon libérable avec ledit orifice de fixation pour positionner l'implant.

9. Appareil selon la revendication 8, dans lequel :
les cylindres définis dans le corps d'implant comprennent des cylindres asservis ;
ledit appareil comprend en outre au moins un maître-cylindre à distance pour fournir un fluide sous pression auxdits cylindres asservis ; et
ledit outil d'insertion peut coopérer avec l'orifice d'alimentation hydraulique et comporte un passage pour délivrer le fluide hydraulique sous pression depuis ledit maître-cylindre.

10. Appareil selon la revendication 9, dans lequel ledit outil d'insertion peut coopérer avec l'orifice d'alimentation en matériau de greffon osseux et comporte un passage pour délivrer un matériau de greffon osseux audit orifice depuis une alimentation à distance dudit matériau.

11. Appareil selon la revendication 10, dans lequel lesdits orifices sont disposés sur une face proximale du corps d'implant, espacés latéralement.

12. Appareil selon la revendication 11, dans lequel une face distale du corps d'implant comprend un bord avant rétréci pour faciliter l'insertion et la mise en place du corps d'implant dans l'espace intervertébral.
